# EUROPÄISCHE PATENTSCHRIFT

(11) **EP 0 858 986 B1**
(45) Veröffentlichungstag und Bekanntmachung des Hinweises auf die Patenterteilung: **31.10.2001**
(21) Anmeldenummer: 98102074.6
(22) Anmeldetag: 06.02.1998
(51) Int. Cl.: C07B 37/08, C07C 403/12, C07C 403/20

(54) **Isomerisierungsverfahren**
Isomerization process
Procédé d'isomérisation

(30) Priorität: 14.02.1997 EP 97102394
(43) Veröffentlichungstag der Anmeldung: 19.08.1998
(73) Patentinhaber: F. HOFFMANN-LA ROCHE AG, 4070 Basel (CH)
(72) Erfinder: Wildermann, Angela, 79713 Bad Säckingen (DE)
(74) Vertreter: Kellenberger, Marcus, Dr.

(56) Entgegenhaltungen:
- FR-A- 2 245 626
- FR-A- 2 291 191
- DATABASE WPI Section Ch, Week 9308 Derwent Publications Ltd., London, GB; Class E17, AN 93-061863 XP002065884 & JP 05 009 482 A (MIYOSHI YUSHI KK)
- KIRMSE: "Methoden der Organischen Chemie", GEORG THIEME VERLAG, STUTTGART, , Band V, Nr. 1B, Seiten 670 - 671

## Beschreibung

Die vorliegende Erfindung betrifft ein neues Verfahren zur katalysierten Isomerisierung von Vitamin A-Verbindungen mit Stickstoffmonoxid als Isomerisierungskatalysator. Insbesondere betrifft die Erfindung die Isomerisierung unerwünschter Isomerer von Vitamin A-Verbindungen, z.B. der 9-Z-, 11-Z-, 9,13-di-Z- und 11,13-di-Z-Vitamin A-Verbindungen im einzelnen oder als Gemische dieser Isomeren, in ein entsprechendes Gemisch der nützlichen all-E- und 13-Z-Verbindungen, die sich zwangsläufig im Gleichgewicht befinden.

In natürlichem Vitamin A, wie es in vielen Fischleberölen, z.B. Suppenflossenhai-, Dorsch-, Heilbutt- und kalifornischem Judenfischleberöl, enthalten ist, besteht der Gesamtgehalt an Vitamin A zu etwa 65% aus all-E-Vitamin A und zu etwa 35% aus 13-Z-Vitamin A. Das all-E-Vitamin A bzw. dessen Alkanoylester besitzen von allen Isomeren die weitaus grösste biologische Wirksamkeit und werden somit nahezu ausschliesslich in der menschlichen und tierischen Ernährung verwendet. Andererseits spielen auch die 13-Z-Vitamin A-Verbindungen eine wichtige Rolle, und zwar als pharmazeutische Wirkstoffe.

Bei den zur Zeit im Handel befindlichen Vitamin A-Präparaten handelt es sich praktisch ausschliesslich um synthetisch hergestellte. Da die bisher bekannten und angewandten Verfahren zur Herstellung einer Vitamin A-Verbindung keine reine all-E-Verbindung, sondern lediglich Gemische von verschiedenen Isomeren mit mehr oder weniger grossen Anteilen des all-E-Isomeren liefern, stellte sich bisher immer das Problem der Isomerisierung der verschiedenen unerwünschten Isomeren in das all-E-Isomere. Das Problem stellte sich einmal, um möglichst hohe Ausbeuten an all-E-Verbindungen zu erzielen und auch - da die totale Z → E-Umwandlung nicht möglich ist - um Gemische zu erhalten, aus welchen das all-E-Isomere auf möglichst einfache Weise isoliert werden kann. Die bisher am meisten angewandte Methode war die Isomerisierung mit Iod in Gegenwart von Pyridin [siehe beispielsweise die Deutsche Auslegeschrift (DAS) 1 468 798]. Die Zugabe von Pyridin ist notwendig, um die Bildung des 9-Z-Isomeren möglichst gering zu halten. Diese Methode besitzt jedoch den Nachteil, dass das Iod nach der Isomerisierung aus dem Reaktionsgemisch und vor der Isolierung der all-E-Verbindung möglichst vollständig entfernt werden muss. Dies geschieht üblicherweise durch Zugabe eines Iod-Reduktionsmittels, wie beispielsweise Natriumthiosulfat, Natriumbisulfit oder Natriumborhydrid, dessen Überschuss man anschliessend durch Waschen, Filtrieren oder andere geeignete Methoden entfernt. Ebenso bekannt ist die photochemische Isomerisierung unter Verwendung von Sensibilisatoren (siehe beispielsweise DAS 2 210 800). Diese Methode ist jedoch mit dem Nachteil behaftet, dass nach Beendigung der Isomerisierung der Sensibilisator entfernt werden muss. Darüber hinaus erfordert die photochemische Isomerisierung (sowohl mit als auch ohne Sensibilisatoren) aufwendige und teure Spezialapparate, was insbesondere beim Arbeiten in grosstechnischem Massstab zu erheblichen Schwierigkeiten führen kann.

Die französische Patentpublikation 2 245 626 offenbart ein Verfahren zur Isomerisierung von Vitamin A und dessen Derivaten in die all-trans (all-E)-, 9-cis(9-Z)- und 13-cis(13-Z)-Isomeren unter Verwendung von Palladium oder einer Palladium enthaltenden Verbindung. Der grösste Anteil des jeweiligen Produktes besteht aus dem all-E-Isomeren, und etwa ein Viertel bis ein Drittel des Produktes besteht aus dem 9-Z-Isomeren; nur sehr wenig des jeweiligen 13-Z-Isomeren wird erhalten. Ziel dieses bekannten Verfahrens ist es, möglichst viel all-E- Isomer aus einem Isomerengemisch von Vitamin A oder einem Derivat davon zu erhalten, wobei unter gewissen Umständen ein beträchtlicher Anteil am 9-Z-Isomeren nicht unerwünscht ist.

Die japanische Patentpublikation (Kokai) 5-009482 offenbart ein Oel zur Metallverarbeitung, welches einen höheren Fettsäureester enthält. Dieser Ester wird aus einem aliphatischen Alkohol und einer Fettsäure hergestellt, welche 0,5 bis 30% einer mindestens 20 Kohlenstoffatome und eine "trans-Form"-Doppelbindung aufweisenden Fettsäure ("trans-Säure") enthält. Man stellt die trans-Säure selber durch katalytische Isomerisierung der entsprechenden cis-Säure her, wobei als Isomerisierungskatalysator u.a. Stickstoffmonoxid verwendet werden kann. Die Isomerisierung wird bei eher höheren Temperaturen durchgeführt, und zwar bei 160 bis 250°C. Auf diese Weise wird typisch eine Isomerisierungsrate von etwa 50 - 75% erzielt.

Ziel der vorliegenden Erfindung ist es, ausgehend von den reinen Isomeren oder auch beliebigen Isomerengemischen, auch in Anwesenheit von Verunreinigungen, ein Gemisch mit einem möglichst hohen Gehalt an der entsprechenden all-E-Vitamin A-Verbindung zu erhalten, aus welchem dann das all-E-Isomere und das zwangsläufig mit diesem Isomeren im

Gleichgewicht befindliche 13-Z-Isomere leicht und ohne grossen Aufwand in möglichst reiner Form isoliert werden können. Es wurde nun überraschenderweise gefunden, dass dieses Ziel durch Katalyse mit gasförmigem Stickstoffmonoxid (NO), insbesondere bei Atmosphärendruck oder "unter leichtem Überdruck," d;h; bei Drücken bis 10 bar (1 MPa) Überdruck, erreicht werden kann. Der Isomerisierungskatalysator NO wird durch Austausch der NO-Atmosphäre oder einfach durch Entspannen des Reaktionsgemisches entfernt. Die Isolierung des all-E- und des 13-Z-Isomeren kann dann nach an sich bekannten Methoden, z.B. Kühlungs- oder Verdampfungskristallisation, erfolgen. Durch die Anwendung dieses Isomerisierungsverfahrens wird ein sehr stabiles Produkt erzeugt. Es sind keine Spezialapparate erforderlich.

Gegenstand der vorliegenden Erfindung ist somit ein Verfahren zur katalysierten Isomerisierung eines Z-Isomeren einer Vitamin A-Verbindung der unten stehenden allgememen Formel I oder eines Gemisches mehrerer solcher Isomeren in ein Gemisch des entsprechenden all-E- und 13-Z-Isomeren dieser Vitamin A-Verbindung, welches dadurch gekennzeichnet ist, dass zur Isomerisierung Stickstoffmonoxid oder ein Stickstoffmonoxid enthaltendes Gasgemisch als Katalysator eingesetzt wird.

Unter dem Ausdruck "Vitamin A-Verbindungen" sind im Rahmen der vorliegenden Erfindung Vitamin A selber (Retinol), Vitamin A-Aldehyd (Retinol) und Vitamin A-Säure und deren Derivate, z.B. Ester, Acetale und Amide, zu verstehen. Der Ausdruck umfasst insbesondere die nachfolgenden Verbindungen der allgemeinen Formel I, wobei keine Angabe der Stereoisomerie angegeben ist: 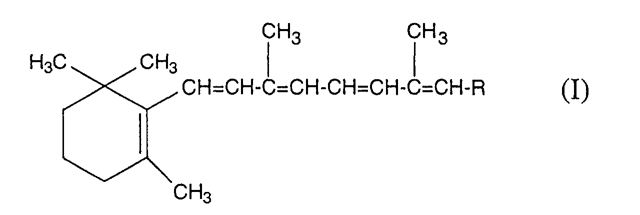 worin R die Gruppe -CHO, -CH₂OH, -COOH; -CH(R¹)₂, -CH₂OR², -COOR³, -CONHR⁴ oder -CON(R⁴)₂ darstellt, worin beide R¹ unabhängig voneinander C₁₋₆-Alkoxy oder zwei Reste R¹ zusammen C₁₋₆-Alkylendioxy, R² Alkanoyl oder Aroyl, R³ Alkyl, Aryl oder Aralkyl und R⁴ bzw. beide R⁴ unabhängig voneinander Wasserstoff, Alkyl, Aryl oder Aralkyl bedeuten.

Im Rahmen der obigen Definition bedeutet der Ausdruck C₁₋₆-Alkoxy" eine Alkoxygruppe mit 1 bis 6 Kohlenstoffatomen, wie beispielsweise Methoxy, Ethoxy oder Propoxy. "C₁₋₆-Alkylendioxy" bedeutet eine Alkylendioxy-Gruppe, die ebenfalls 1 bis 6 Kohlenstoffatome enthält, z.B. Methylendioxy oder Ethylendioxy. In beiden Fällen kann je nach Anzahl der Kohlenstoffatome der Alkyl- bzw. Alkylenteil geradkettig oder verzweigt sein. Der Ausdruck "Alkanoyl" umfasst sowohl geradkettige als auch verzweigte Alkanoylgruppen mit 1-18 Kohlenstoffatomen, wie beispielsweise Formyl, Acetyl, Propionyl, Butyryl, Stearoyl und Palmitoyl. Der Ausdruck "Aroyl" leitet sich von den aromatischen Carbonsäuren mit 7 und 11 Kohlenstoffatomen ab und bedeutet demgemäss Benzoyl bzw. Napthoyl. Der Ausdruck "Alkyl" bedeutet eine geradkettige oder verzweigte

Alkylgruppe mit 1 bis 18 Kohlenstoffatomen, z.B. Methyl, Ethyl, Propyl, Butyl, Decyl, Dodecyl, Hexadecyl oder Octadecyl. Der Ausdruck "Aryl" als solches oder als Teil von "Aralkyl" bedeutet insbesondere Phenyl oder Naphthyl. Schliesslich umfasst der Ausdruck "Aralkyl" solche Gruppen mit 1 bis 4 Kohlenstoffatomen im aliphatischen Teil, z.B. Benzyl und Phenylpropyl.

Vorzugsweise verwendet man als Vitamin A-Verbindung der Formel I Vitamin A-Acetat oder -Säure.

Ein zu isomerisierendes Z-Isomeres einer Vitamin A-Verbindung der Formel I, z.B. das 9-Z-, das 11-Z-, das 9,13-di-Z- oder das 11,13-di-Z-Isomere, kann allein oder als Bestandteil eines Gemisches mehrerer solcher Z-Isomeren isomerisiert werden, wobei in einem Gemisch auch bereits einigermassen das all-E-Isomere und das zwangsläufig mit diesem all-E-Isomeren im Gleichgewicht befindliche 13-Z-Isomere und/oder Verunreinigungen vorhanden sein können. Darüber hinaus können mehrere Vitamin A-Verbindungen als Isomere in einem zu isomerisierenden Gemisch vorhanden sein. Ein typisches Beispiel eines solchen Gemisches ergibt sich aus der mehrstufigen Produktion einer Vitamin A-Verbindung, z.B. Vitamin A-Acetat: Nach mehreren Stufen entsteht durch Kristallisation und Filtration ein eine all-E-Vitamin A-Verbindung enthaltendes Kristallisat und eine Mutterlauge. Diese Mutterlauge enthält neben den Verunreinigungen sowohl das all-E-Isomere als auch Z-Isomere der erwünschten Vitamin A-Verbindung, wobei der Anteil an isomerisierbaren Z-Isomeren beträchtlich sein mag. Dieses Gemisch, oder die nach Entfernung des Grossteils der Verunreinigungen verbleibende Mutterlauge, kann dem erfindungsgemässen Isomerisierungsverfahren unterworfen werden. Nach Durchführung des erfindungsgemässen Isomerisierungsverfahrens und Entfernung des all-E-Isomeren entsteht ein am 13-Z-Isomeren reiches Gemisch, aus welchem gewünschtenfalls das 13-Z-Isomere ebenfalls entfernt werden kann.

Die erfindungsgemäss katalysierte Isomerisierung erfolgt zweckmässigerweise in einem inerten Lösungsmittel, selbst dann, wenn das zu isomerisierende Isomere oder Isomerengemisch bei der Verfahrenstemperatur flüssig ist. Als Lösungsmittel kommen in Frage sowohl polare organische Lösungsmittel, z.B. Acetonitril und Dimethylformamid (aprotische polare Lösungsmittel) als auch apolare organische Lösungsmittel, wie aliphatische und aromatische Kohlenwasserstoffe, z.B. Pentan, Hexan, Heptan, Benzol, Toluol, Xylol und Petrolether; und halogenierte aliphatische und aromatische Kohlenwasserstoffe, z.B. Methylenchlorid, Ethylenchlorid, Chloroform, Tetrachlorkohlenstoff und Chlorbenzol. Weitere in Frage kommende polare organische Lösungsmittel sind niedere aliphatische Alkohole, z.B. Methanol, Ethanol und Propanol (protische polare Lösungsmittel), und niedere aliphatische Ester, z.B. Essigsäure-methylester und Essigsäure-ethylester (aprotische polare Lösungsmittel). Es können auch Lösungsmittelgemische, auch in Kombination mit geringen Mengen Wasser, verwendet werden. Besonders bevorzugte Lösungsmittel sind die aliphatischen Kohlenwasserstoffe, insbesondere Hexan, und die niederen aliphatischen Alkohole, insbesondere Ethanol.

Sofern eine zu isomerisierende Substanz in flüssiger Form vorliegt, kann die Isomerisierung auch ohne Lösungsmittel durchgeführt werden. Die Verwendung eines Lösungsmittels wird jedoch im Hinblick auf eine anschliessende Kristallisation bevorzugt.

Bei der Isomerisierung eingesetzte Lösungen sind zweckmässigerweise solche, deren Konzentration bis etwa 90 Ma.-% (Masse Edukt zu Masse gesamt), insbesondere von etwa 5 Ma.-% bis etwa 80 Ma.-%, beträgt. Besonders bevorzugt sind etwa 50 bis 70 Ma.-%ige Lösungen, da dies für die anschliessende Kristallisation des all-E-Isomeren die besten Bedingungen darstellt.

Der katalytische Kontakt mit NO erfolgt zweckmässigerweise durch Einleiten von NO oder eines NO enthaltenden Gasgemisches in die eventuell gelöste, zu isomerisierende Vitamin A-Verbindung der Formel I und Dispergieren des NO bzw. des NO enthaltenden Gasgemisches, z.B. durch Umwälzen des Gasstroms, bei Atmosphärendruck oder "unter leichtem Überdruck ", d.h. bei Drücken bis 10 bar (1 MPa) Überdruck, insbesondere Drücken von 0,1 bar (10 kPa) Überdruck bis 3 bar (300 kPa) Überdruck. Werden zur Katalyse NO-enthaltende Gasgemische verwendet, enthalten diese zweckmässigerweise 1 bis etwa 90 Ma.-% NO (Masse NO zu Masse Gemisch). Bevorzugt werden Gasgemische mit 10 bis 80 Ma.-% NO verwendet, insbesondere solche mit 10 bis 60 Ma.-% NO. Zur Herstellung des NO-Gasgemisches eignen sich inerte Gase, wie beispielsweise Stickstoff, Helium, Argon, Kohlendioxid, Distickstoffmonoxid, Methan und Ethan. Bevorzugt wird Stickstoff verwendet, welcher gleichzeitig zur Inertisierung des Reaktionsgemisches eingesetzt wird.

Das erfindungsgemässe Isomerisierungsverfahren kann zweckmässigerweise bei Temperaturen bis 200°C durchgeführt werden. Vorzugsweise erfolgt das Verfahren bei Temperaturen bis 150°C, insbesondere bis 100°C. Besonders bevorzugt erfolgt das Verfahren bei Temperaturen im Bereich von 30 bis 80°C. Auch bei Raumtemperatur und darunter kann die Isomerisierung erfolgen. Zweckmässigerweise werden bei der Isomerisierung die Konzentration und Temperatur so gewählt, dass das gebildete all-E-Isomere kontinuierlich abgeschieden wird; dies kann auf an sich bekannte Weise erfolgen.

Die Isomerisierungsdauer liegt im allgemeinen zwischen 1 Minute und 50 Stunden, insbesondere zwischen 10 Minuten und 30 Stunden, vorzugsweise zwischen 30 Minuten und 20 Stunden. Die Isomerisierungsdauer ist weitgehend abhängig von der gewählten Temperatur und dem eingestellten NO-Druck. Niedrige Temperaturen und niedrige NO-Drücke haben bei langer Isomerisierungsdauer denselben Effekt wie hohe Temperaturen und entsprechend kürzere Isomerisierungsdauer. Da bekanntlich Vitamin A-Verbindungen ziemlich instabile Verbindungen sind, werden die Reaktionsbedingungen zweckmässig so gewählt, dass das Reaktionsgemisch nicht für längere Dauer auf höhere Temperaturen erhitzt werden muss.

Die Isomerisierung erfolgt zweckmässig unter Luftausschluss, also unter Inertgas, z.B. Stickstoff oder Argon. Weiterhin kann die Isomerisierung sowohl diskontinuierlich als auch kontinuierlich durchgeführt werden.

Nach Beendigung des Isomerisierungsverfahrens kann die Isolierung der gewünschten all-E- bzw. 13-Z-Vitamin A-Verbindung erfolgen, und zwar nach an sich bekannten Methoden, wie beispielsweise Kühlungs- oder Verdampfungskristallisation. Dabei sind für eine möglichst vollständige Abtrennung die deutlichen Unterschiede im Löslichkeitsverhalten zwischen den zu isolierenden Isomeren und den restlichen Isomeren (einschliesslich Verunreinigungen) von Bedeutung.

Das erfindungsgemässe Isomerisierungsverfahren wird durch die nachfolgenden Beispiele veranschaulicht; in den Beispielen sind sämtliche angegebenen Analysen durch Hochdruckflüssigchromatographie (HPLC) durchgeführt worden.

### Beispiel 1

In einen Sulfierkolben werden 49 g eines Gemisches enthaltend 23% all-E-Vitamin A-Acetat, 39% 13-Z-Vitamin A-Acetat, 18% 11-Z-Vitamin A-Acetat, 5% 11,13-di-Z-Vitamin A-Acetat und 3% 9-Z- und/oder 9,13-di-Z-Vitamin A-Acetat (Rest Verunreinigungen) unter Argon in 35 ml Essigsäure-methylester gelöst. In diese Lösung wird nun unter Rühren 10 Minuten lang reines NO-Gas durchgeleitet. Anschliessend wird die Reaktionsapparatur vollständig geschlossen und die Lösung während 5 Stunden bei Raumtemperatur intensiv gerührt. Anschliessend wird die NO-Atmosphäre durch Inertgas ausgetauscht. Man erhält ein Gemisch enthaltend 43% all-E-, 26% 13-Z-, 12% 11-Z-, 4% 11,13-di-Z- und 3% 9-Z-und/oder 9,13-di-Z-Vitamin A-Acetat.

### Beispiel 2

In einer druckbeständigen Apparatur werden 200 g eines Gemisches enthaltend 23% all-E-, 40% 13-Z-, 17% 11-Z-, 5% 11,13-di-Z- und 3% 9-Z-und/oder 9,13-di-Z-Vitamin A-Acetat unter Stickstoff in 140 ml Hexan gelöst. Nachdem die Apparatur bei Atmosphärendruck mit Stickstoff überlagert ist, wird NO-Gas bei Raumtemperatur unter Rühren bis zu einem Überdruck von 0,5 bar (50 kPa) aufgedrückt und die Apparatur verschlossen. Die Lösung wird unter Rühren auf 40°C erwärmt und während 5 Stunden intensiv gerührt. Anschliessend wird die Apparatur entspannt und auf Raumtemperatur abgekühlt. Man erhält ein Gemisch enthaltend 48% all-E-, 24% 13-Z-, 4% 11-Z-, 4 % 11,13-di-Z- und 3% 9-Z-und/oder 9,13-di-Z-Vitamin A-Acetat.

### Beispiel 3

In einer druckbeständigen Apparatur werden 100 g eines Gemisches enthaltend 23% all-E-, 32% 13-Z-, 15% 11-Z-, 6% 11,13-di-Z- und 3% 9-Z-und/oder 9,13-di-Z-Vitamin A-Acetat ohne Lösungsmittel unter Stickstoff vorgelegt. Nachdem die Apparatur bei Atmosphärendruck mit Stickstoff überlagert ist, wird NO-Gas bei Raumtemperatur unter Rühren bis zu einem Überdruck von 0,5 bar (50 kPa) aufgedrückt und die Apparatur verschlossen. Die Flüssigkeit wird unter Rühren auf 40°C erwärmt und während 5 Stunden intensiv gerührt. Anschliessend wird die Apparatur entspannt und auf Raumtemperatur abgekühlt. Man erhält ein Gemisch enthaltend 48% all-E-, 21% 13-Z-, 2% 11-Z-, 5 % 11,13-di-Z- und 3% 9-Z-und/oder 9,13-di-Z-Vitamin A-Acetat.

### Beispiel 4

In einer druckbeständigen Apparatur werden 19 g eines Gemisches enthaltend 94% all-E-Vitamin A-Acetat und 3% 13-Z-Vitamin A-Acetat unter Stickstoff in 38 g Ethanol gelöst. Nachdem die Apparatur bei Atmosphärendruck mit Stickstoff überlagert ist, wird NO-Gas bei Raumtemperatur unter Rühren bis zu einem Überdruck von 0,5 bar (50 kPa) aufgedrückt und die Apparatur verschlossen. Die Lösung wird unter Rühren auf 40°C erwärmt und während 5 Stunden intensiv gerührt. Anschliessend wird die Apparatur entspannt und auf Raumtemperatur abgekühlt. Man erhält ein Gemisch enthaltend 67% all-E-Vitamin A-Acetat und 28% 13-Z-Vitamin A-Acetat. Der Gehalt der anderen Isomeren (11-Z-, 11,13-di-Z- und 9-Z- und/oder 9,13-di-Z-Vitamin A-Acetat) liegt deutlich unter 1%.

## Patentansprüche

1. Verfahren zur katalysierten Isomerisierung eines Z-Isomeren einer Vitamin A-Verbindung der allgemeinen Formel 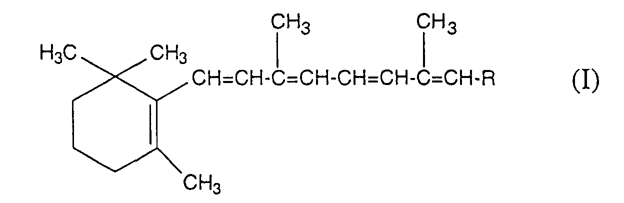 worin R die Gruppe -CHO, -CH₂OH, -COOH; -CH(R¹)₂, -CH₂OR², -COOR³, -CONHR⁴ oder -CON(R⁴)₂ darstellt, worin beide R¹ unabhängig voneinander C₁₋₆-Alkoxy oder zwei Reste R¹ zusammen C₁₋₆-Alkylendioxy, R² Alkanoyl oder Aroyl, R³ Alkyl, Aryl oder Aralkyl und R⁴ bzw. beide R⁴ unabhängig voneinander Wasserstoff, Alkyl, Aryl oder Aralkyl bedeuten, oder eines Gemisches mehrerer solcher Isomeren in ein Gemisch des entsprechenden all-E- und 13-Z-Isomeren dieser Vitamin A-Verbindung, **dadurch gekennzeichnet, dass** zur Isomerisierung Stickstoffmonoxid oder ein Stickstoffmonoxid-enthaltendes Gasgemisch als Katalysator eingesetzt wird.

2. Verfahren nach Anspruch 1, **dadurch gekennzeichnet, dass** es sich bei den zu isomerisierenden Z-Isomeren um das 9-Z-, das 11-Z-, das 9,13-di-Z- oder das 11,13-di-Z-Isomere oder um Gemische davon handelt.

3. Verfahren nach Anspruch 1 oder 2, **dadurch gekennzeichnet, dass** die Isomerisierung in einem polaren oder apolaren organischen Lösungsmittel erfolgt.

4. Verfahren nach Anspruch 3, **dadurch gekennzeichnet, dass** das Lösungsmittel ein aliphatischer Kohlenwasserstoff, Methand, Ethanol oder Propanol ist.

5. Verfahren nach Anspruch 4, **dadurch gekennzeichnet, dass** das Lösungsmittel Hexan bzw. Ethanol ist.

6. Verfahren nach einem der Ansprüche 1 bis 5, **dadurch gekennzeichnet, dass** der katalytische Kontakt mit Stickstoffmonoxid durch Einleiten von Stickstoffmonoxid oder eines Stickstoffmonoxid enthaltenden Gasgemisches in die eventuell gelöste, zu isomerisierende Vitamin A-Verbindung und Dispergieren des Stickstoffmonoxids bzw. des Stickstoffmonoxid enthaltenden Gasgemisches bei Atmosphärendruck oder bis zu 1MPa Überdruck erfolgt.

7. Verfahren nach Anspruch 6, **dadurch gekennzeichnet, dass** der Ueberdruck 0,1 bis 3 bar (10 bis 300 kPa) beträgt.

8. Verfahren nach einem der Ansprüche 1 bis 7, **dadurch gekennzeichnet, dass** zur Katalyse ein Gemisch aus Stickstoffmonoxid und Stickstoff, vorzugsweise eines mit 10 bis 60 Ma.-% Stickstoffmonoxid, verwendet wird.

9. Verfahren nach einem der Ansprüche 1 bis 8, **dadurch gekennzeichnet, dass** es bei Temperaturen im Bereich von 30 bis 80°C erfolgt.

10. Verfahren nach einem der Ansprüche 1 bis 9, **dadurch gekennzeichnet, dass** man als Verbindung der Formel I. Vitamin A-Acetat oder -Säure verwendet.

## Claims

1. A process for the catalyzed isomerization of a Z isomer of a vitamin A compound of the general formula 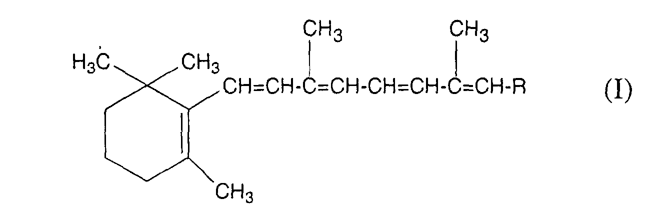 wherein R represents the group -CHO, -CH₂OH, -COOH, -CH(R¹)₂, -CH₂OR², -COOR³, -CONHR⁴ or -CON(R⁴)₂, in which both R¹s each independently signify C₁₋₆-alkoxy or two residues R¹ together signify C₁₋₆-alkylenedioxy, R² signifies alkanoyl or aroyl, R³ signifies alkyl, aryl or aralkyl and R⁴ or both R⁴s each independently signify hydrogen, alkyl, aryl or aralkyl,
or of a mixture of several such isomers into a mixture of the corresponding all-E and 13-Z isomers of this vitamin A compound, which process comprises using nitrogen monoxide or a gas mixture containing nitrogen monoxide as the catalyst for the isomerization.

2. A process according to claim 1, wherein the Z isomer to be isomerized is the 9-Z, the 11-Z, the 9,13-di-Z or the 11,13-di-Z isomer or a mixture thereof.

3. A process according to claim 1 or 2, wherein the isomerization is effected in a polar or apolar organic solvent.

4. A process according to claim 3, wherein the solvent is an aliphatic hydrocarbon, methanol, ethanol or propanol.

5. A process according to claim 4, wherein the solvent is hexane or ethanol.

6. A process according to any one of claims 1 to 5, wherein the catalytic contact with nitrogen monoxide is effected by introducing nitrogen monoxide or a gas mixture containing nitrogen monoxide into the optionally dissolved vitamin A compound to be isomerized and dispersing the nitrogen monoxide or the gas mixture containing nitrogen monoxide at atmospheric pressure or up to 1MPa over-pressure.

7. A process according to claim 6, wherein the over-pressure amounts to 0.1 to 3 bar (10 to 300 kPa).

8. A process according to any one of claims 1 to 7, wherein a mixture of nitrogen monoxide and nitrogen, preferably one with 10 to 60 wt.% nitrogen monoxide, is used for the catalysis.

9. A process according to any one of claims 1 to 8, wherein the isomerization is effected at temperatures in the range of 30°C to 80°C.

10. A process according to any one of claims 1 to 9, wherein vitamin A acetate or vitamin A acid is used as the compound of formula I.

## Revendications

1. Procédé d'isomérisation catalysée d'un isomère Z d'un composé de vitamine A de la formule générale : 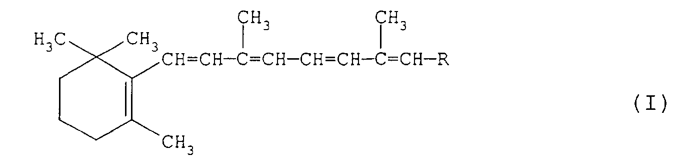 dans laquelle R est le groupe -CHO, -CH₂OH, -COOH, -CH(R¹)₂, -CH₂OR², -COOR³, -CONHR⁴ ou -CON(R⁴)₂, où les deux R¹ sont indépendamment l'un de l'autre alcoxy en C₁ à C₆ ou deux résidus R¹ ensemble sont alkylènedioxy en C₁ à C₆ ; R² est alcanoyle ou aroyle ; R³ est alkyle, aryle ou aralkyle ; et R⁴ ou les deux R⁴ sont indépendamment l'un de l'autre hydrogène, alkyle, aryle ou aralkyle ou d'un mélange de plusieurs de ces isomères en un mélange des isomères tout E et 13-Z correspondants dudit composé de vitamine A, **caractérisé en ce que** l'on utilise du monoxyde d'azote ou un mélange gazeux contenant du monoxyde d'azote comme catalyseur pour l'isomérisation.

2. Procédé selon la revendication 1, **caractérisé en ce que** les isomères Z à isomériser sont les isomères 9-Z, 11-Z, 9,13-di-Z ou 11,13-di-Z ou un mélange de ceux-ci.

3. Procédé selon la revendication 1 ou la revendication 2, **caractérisé en ce que** l'isomérisation s'effectue dans un solvant organique polaire ou apolaire.

4. Procédé selon la revendication 3, **caractérisé en ce que** le solvant est un hydrocarbure aliphatique, le méthanol, l'éthanol ou le propanol.

5. Procédé selon la revendication 4, **caractérisé en ce que** le solvant est l'hexane ou l'éthanol.

6. Procédé selon l'une des revendications 1 à 5, **caractérisé en ce que** le contact catalytique avec le monoxyde d'azote se fait en introduisant du monoxyde d'azote ou un mélange gazeux contenant du monoxyde d'azote dans le composé de vitamine A dissous à isomériser éventuellement et en dispersant le monoxyde d'azote ou le mélange gazeux contenant du monoxyde d'azote à la pression atmosphérique ou à une pression positive allant jusqu'à 1 MPa.

7. Procédé selon la revendication 6, **caractérisé en ce que** la pression positive est de 0,1 à 3 bars (10 à 300 kPa).

8. Procédé selon l'une des revendications 1 à 7, **caractérisé en ce que** l'on utilise pour la catalyse un mélange de monoxyde d'azote et d'azote, de préférence un mélange contenant de 10 à 60 % en masse de monoxyde d'azote.

9. Procédé selon l'une des revendications 1 à 8, **caractérisé en ce qu'**il s'effectue à des températures dans la plage de 30 à 80°C.

10. Procédé selon l'une des revendications 1 à 9, **caractérisé en ce que** l'on utilise comme composé de la formule I l'acétate de vitamine A ou l'acide.
